# EUROPEAN PATENT SPECIFICATION

(11) **EP 2 164 614 B1**
(45) Date of publication and mention of the grant of the patent: **19.04.2017**
(21) Application number: 08771434.1
(22) Date of filing: 19.06.2008
(51) Int. Cl.: A61M 5/165, B01D 15/00, B01D 15/08, B01D 15/32, B01J 20/32, B01J 20/10, B01J 20/26

(54) **METHODS FOR DYNAMIC FILTRATION OF PHARMACEUTICAL PRODUCTS**
VERFAHREN ZUR DYNAMISCHEN FILTRATION VON PHARMAZEUTISCHEN PRODUKTEN
PROCÉDÉS POUR LA FILTRATION DYNAMIQUE DE PRODUITS PHARMACEUTIQUES

(30) Priority: 22.06.2007 US 766881
(43) Date of publication of application: 24.03.2010
(73) Proprietor: General Electric Company, Schenectady, NY 12345 (US)
(72) Inventor: LEACH, Andrew Michael, Clifton Park, New York 12065 (US); MILLER, Peter, New London, Connecticut 06320 (US); TELFEYAN, Eric John, Guilderland, New York 12084 (US); WHITT, David Brandon, Albany, New York 12209 (US)
(74) Representative: Livgard, Kim Are Birkeli
(86) International application number: PCT/US2008/067440
(87) International publication number: WO 2009/002794

(56) References cited:
- JP-A- 2000 351 799
- US-A- 5 091 080
- US-A1- 2002 110 495

## Description

### BACKGROUND

The invention relates generally to methods for filtering pharmaceutical products.

During a pharmaceutical compounding process one or more chemical components are required to be removed from the end product for safety or efficacy purposes. As used herein, the term "chemical components" refers to one or more chemicals present in the initial mixture used to prepare the pharmaceutical product. The chemical components may include any form of compounds intentionally added to, or present from material impurities, or present from contamination of an unrefined pharmaceutical product that are not desired in the end pharmaceutical product. Various methods of filtration and separation are known in the art for removing - chemical components from a mixture to obtain a pharmaceutical product that is suitable to be administered to a subject. The conventional removal methods typically require use of solvents or materials that unintentionally add further matter to the mixture that is not desired in the final pharmaceutical product. The undesired matter needs to be removed from the device before conducting the filtration process. This undesired matter may include solvents that are used to prepare the device to perform the filtration or separation or solvents or materials added to the mixture. Alternatively, this undesired matter may also include biologically relevant materials such as endotoxins, proteins, nucleic acids, bacteria or viruses that are contained within the conditioning system or solvents and are subsequently introduced to the filtration device. The additional steps involving addition and subsequent removal of solvents and other materials from the device or the mixture adds to the complexity of the filtration process and to the complexity of the process equipment. Further, these additional steps result in longer time duration for the overall filtration process. Furthermore, quality control mechanisms are required to ensure that the undesired matter is not carried in to the pharmaceutical product. In addition, there is a chance of contamination of the mixture due to extraneous material entering the filtering device while carrying out the steps of administering and removing the undesired matter from the device.

Therefore, there is a need to explore new methods and devices for filtration of pharmaceutical products that do not require addition of solvents or other materials to the filtration device or to the pharmaceutical mixture and that are relatively easier to perform. Further, there is a need for new methods and devices that can be pre-conditioned and stored, such that these devices are ready to use for filtration without conducting any further steps for conditioning the device immediately prior to filtration. Furthermore, there is a need for a device that is sealed to prevent any extraneous material from entering the device either before or during filtration, or once the device is prepared and stored for filtration.

### BRIEF DESCRIPTION

In one embodiment, a device for dynamic filtration of a pharmaceutical product is provided. The device includes a resin configured to selectively retain one or more components from a mixture having the pharmaceutical product, where the resin is configured to be activated by a medium of the mixture. The device further includes at least one positioning material disposed adjacent to the resin, where the positioning material is configured to provide mechanical support to the resin to at least partially retain the resin in position.

In another embodiment, a system for filtering pharmaceutical product to be administered in a patient is provided. The system includes a filtering device, a filter body, and a resin disposed within the filter body and configured to filter a mixture of a pharmaceutical product, wherein the mixture comprises a medium such that the medium is configured to *in-situ* activate the resin. The filtering device further includes a positioning material configured to support the resin in the filter body, wherein the filtering device is configured to filter both soluble and insoluble species from the mixture of the pharmaceutical product.

In yet another embodiment, a method of filtering an unrefined pharmaceutical product is provided. The method includes mixing a buffer, a chelator, and one of an acidic medium, a basic medium, or a neutral medium, with the unrefined pharmaceutical product to form a filtering mixture; and passing the filtering mixture through a filtering device.

In yet another embodiment, a device for dynamic filtration of a pharmaceutical product is provided. The device includes a resin configured to selectively retain one or more components from a mixture having the pharmaceutical product, and at least one positioning material disposed adjacent to the resin, where the positioning material is configured to provide mechanical support to the resin to at least partially retain the resin in position. The device is configured to remove both soluble and insoluble components from the mixture comprising the pharmaceutical product.

### DRAWINGS

These and other features, aspects, and advantages of the present invention will become better understood when the following detailed description is read with reference to the accompanying drawings in which like characters represent like parts throughout the drawings, wherein:
FIGS. 1-2 are cross-sectional side views illustrating alternative arrangements of resins and positioning material, in accordance with aspects of the present technique;
FIG. 3 is a perspective view of a diffuser employed in the filter configuration of FIG. 2;
FIGS. 4-8 are schematics illustrating an exemplary filtering devices for filtering of a pharmaceutical product, in accordance with aspects of the present technique; and
FIGS. 9A is a cross-sectional side view illustrating a radial filtration device for filtering of a pharmaceutical product, in accordance with aspects of the present technique;
FIG. 9B is a top view of the radial filtration device of FIG. 9A;
FIG. 10 is a cross-sectional side view of a filtering device having a conical shape at the exit location; and
FIG. 11 is an illustration of an exemplary MRI system and filtering devices; in accordance with aspects of the present technique.

### DETAILED DESCRIPTION

Embodiments of the present technique relate to devices and methods for dynamic filtration of pharmaceutical products to selectively retain one or more components from a mixture of the pharmaceutical product. As used herein, the term "dynamic filtration" refers to the ability of a filtration device to remove or filter one or more components of a mixture having a time varying solubility in a medium of the mixture carrying the pharmaceutical product. As will be described in detail below, the solubility of the components may vary with changing pressure, temperature, volume, pH of the medium, for example. As used herein, the term "pharmaceutical product" includes a compound or a mixture of compounds that are pharmaceutically relevant. The pharmaceutical product may be the end product that is being used. Alternatively, the pharmaceutical product may be an intermediate product formed while making a pharmaceutical compound. The one or more components that are selectively retained by the filtration device are generally impurities or undesired compounds present in the pharmaceutical product that are not desired in the end product. The one or more components may be by-products formed from a reaction involving two or more reactants used to form the pharmaceutical product, for example.

As will be appreciated, typically, a process of filtering a pharmaceutical product requires an additional step of conditioning the filtration device immediately prior to passing the mixture having the pharmaceutical product through the device. In conventional filtration devices, which are employed for filtering a pharmaceutical mixture, conditioning of the device prior to filtering often involves the use of additional chemicals in the form of conditioning agents or solvents. As used herein, the term "conditioning" refers to a modification of one or more parts of the filtration device, such that the one or more parts are configured to retain the impurities or chemical components from the mixture after undergoing the modification. Nonlimiting examples of modification may include wetting a surface of a resin, creating conditions in the resin that allow the chemicals to be filtered to interact with the surface of the resin, or creating a liquid/solid interface from a solid/gas interface. For example, some conventional devices are conditioned using alcohol. The alcohol added for conditioning step needs to be subsequently removed to filter the pharmaceutical product. In addition, the solvent or other materials used for conditioning are typically not desired in the end product, therefore, this undesired matter needs to be removed from the filtration device once the device is conditioned, and before the filtration process begins. The removal of such chemicals requires extra process steps, and hence, more process time.

Further, the conditioning at the point-of-use may cause the potential problem of having biologically relevant materials introduced into the system from the onsite conditioning process (i.e. through solvents and water). Also, in systems requiring conditioning of the filter at the point-of-use, the additional steps of removing the solvent from the filter are integral part of the filtration step and contribute to prolonging the filtration process. The additional steps lead to prolonged process of manufacturing the pharmaceutical product, also, sometimes due to limitation on time, or the number of process steps, or the complexity of the process, it may not be possible to entirely separate out the conditioning agent from the pharmaceutical product, thereby resulting in a contaminated pharmaceutical product. Accordingly, elimination of conditioning step just prior to filtration prevents the administration of these unwanted conditioning agents, prevents the use of additional steps otherwise required to separate out the conditioning agents and other chemicals added to facilitate conditioning of the device prior to filtration, and greatly reduces the risk of introduction of unwanted biologically relevant materials. As will be described in detail below, the filtration devices of the present technique do not require addition of undesired matter either during or just prior to filtration. Accordingly, the filtration devices of the present technique do not require additional steps otherwise employed in conventional filtration devices to remove the earlier administered undesired matter prior to carrying out the filtration of the mixture. In one embodiment, the filtration device is configured to be conditioned during filtration. In another embodiment, the filtration device is pre-conditioned and stored under controlled conditions prior to filtration.

In some embodiments, the filtration device is configured to be conditioned by the medium of the pharmaceutical mixture. As will be described in detail below, in these embodiments, the filtration device includes a resin that is activated by the medium of the mixture during the filtration process. The activated resin is configured to selectively retain one or more components from a mixture comprising the pharmaceutical product. Therefore, by employing the device of the present technique, the additional steps involving conditioning the device prior to filtration become redundant. A combination of resin and medium may be chosen depending on their mutual compatibility. Further, the medium may be chosen such that the medium may be easily separated from the pharmaceutical product. Alternatively, the medium may be chosen such that the medium may be retained in the final pharmaceutical product, without contributing to any deleterious effects to the pharmaceutical product.

The mixture of the pharmaceutical product may include a medium such that the resin is configured to be *in-situ* conditioned by the medium or the filtration mixture. In other words, the device is configured to filter the one or more components from the mixture without being conditioned prior to filtration. In its activated state, the resin is configured to filter out the one or more undesired components from the mixture of the pharmaceutical product by retaining the undesired components and allowing the rest of the mixture to pass through the resin. The resin may be configured for chemical retention, mechanical retention, or both of the one or more components of the pharmaceutical mixture. In certain embodiments, the resin material is a porous material. In these embodiments, the resin material has a higher surface area, and therefore higher chemical filtration efficiency. Characteristics such as particle size, particle shape, and packing efficiency of the resin material may affect the ability of the resin bed to remove particulate. In mechanical filtration, the particle size of the particulates that need to be filtered out are likely much larger than the pore size of the resin materials. In one embodiment, the pore size of the resin is in a range from about 60A° to about 4000A°.

In some embodiments, the resin is adapted with surface modification. The choice of the resin materials may vary depending on whether the resin is configured to be activated by the medium of the mixture, or whether the resin is pre-activated and stored in the device. In embodiments where the resin is configured to be activated by the medium, it is required to have the resin that contains suitable polar/hydrophilic functionalities. For example, the surface of the particulate that comes in contact with the mixture, the bulk (or substrates) of the resin may be modified with one or more non-polar functionalities, or a combination of a non-polar and polar functionalities such that the modified surface can be activated by the medium. Typically, the substrates may be polymer based. The polar functionalities are chosen such that they allow the medium to activate the resin but do not negatively impact the retention of the one or more components by the hydrophobic functionality of the resin. In case of filtration devices where the resin is configured to be activated by the medium of the mixture, the medium of the mixture may be aqueous based. The aqueous based medium may be neutral, basic or acidic in nature. In certain embodiments, the aqueous based medium may change dynamically from acidic to basic, or basic to acidic, or from a neutral pH to either basic or acidic condition, or from an acidic or basic condition to a neutral pH. When the pharmaceutical mixture is passed through the device, one or more of the components are trapped by hydrophobic and/or polar functionalities in the resin and hence, selectively retained. The rest of the mixture is allowed to pass through the device.

In some embodiments, the resins may be activated based on the morphology. The resin may have suitable morphology, such that the resin can be wet by an aqueous based medium, for example. As will be appreciated, it is desirable for the resin to have hydrophobic character to retain the impurities, and be compatible with the mixture to be filtered. In certain embodiments, the resins may include pores that are of the sizes such that upon application of pressure, the pores allow the medium of the pharmaceutical mixture to wet the resin surface allowing interaction of the chemical components to be filtered from the pharmaceutical mixture with the resin's functionality. In these embodiments, the pores of the resin may be wet by the medium even in the absence of hydrophilic moieties on the resin. In one embodiment, pressure is used in addition to large pore size resin to achieve wetting. The pressure in this case is the fluidic pressure of the pharmaceutical mixture being introduced into the device. In an exemplary embodiment, a flow rate of the mixture at the filtering device is in a range from about 3 mL/s to about 12 mL/s. In another embodiment, an additional pressure may be required to activate the resin.

In embodiments where the filtration device is pre-conditioned and stored for later use, the filtration device is pre-conditioned with a solvent, such as an alcohol, to activate the resin, the filter is then flushed with water to remove the solvent. In one embodiment, ethanol may be used as the solvent for conditioning the filter. The advantage of employing ethanol is that usually the pharmaceutical product has tolerance levels for ethanol. The pre-conditioned filtration devices may be directly used at the time of filtration without having to conduct any further steps related to conditioning, thereby reducing the overall time for filtration. In these embodiments, the resin is activated and stored in the device prior to filtration. The resin may be activated using solvents or other conditioning materials. If the solvents or the other conditioning materials are not desired in the end product, the filtration device may be processed or washed to remove the solvents or the other conditioning materials from the device. In certain embodiments, the pre-conditioned device is preserved by bringing the temperature of the device below the freezing point of the solvent left in the device. For example, in case of hydrophobic resins because a de-wetting process occurs where the water is expelled from the pores over time and hence the resin may loose its efficiency, freezing the solvent facilitates storing the filters with solvent in them. In case of pre-conditioned filters, the additional steps required to separate out the conditioning chemicals/solvent from the filter prior to filtration are carried out before storing the device. Hence, these additional steps do not contribute to prolonging the filtration process.

In some embodiments, the resin may be activated during the manufacturing of the filtration device. Once the device is conditioned, in some cases the solvents are removed, the device may then be sealed to prevent contaminants, such as biologically relevant materials, from entering the system. In embodiments where water acts as the solvent, it may not be required to remove the solvents prior to sealing the device. Once conditioned and stored, the pre-conditioned device acts as a ready-to-use device that may be directly used for filtration without performing any conditioning steps prior to filtration. In one embodiment, the filtration device may be sterilized to remove any biologically relevant material, for example. In one embodiment, the pre-conditioned device is hermetically sealed to retain the sterility prior to and during filtration. It should be noted that similar to pre-conditioned devices, the filtration devices where the resin is configured to be *in-situ* conditioned by the medium or the filtration mixture may be hermetically sealed to prevent any extraneous material from entering the device either before or during filtration. The pre-conditioned filter works with many different resins that have a hydrophobic character. The resin retains the one or more components and is compatible with the medium/mixture to be filtered. Examples of such resins include Silica based ¹⁸C used in solid phase extraction, or resins employed in flash chromatography applications.

Further, the device includes at least one positioning material disposed adjacent to the resin, where the positioning material is configured to provide mechanical support to the resin to at least partially retain the resin in position. Additionally, the positioning material may be configured to at least partially retain one or more components from the pharmaceutical mixture. The positioning material may be made of a material that is inert to any solvents, medium, or pharmaceutical components in the mixture. Further, for healthcare application, the positioning material must be suitable for use in medical devices where it comes into contact with a pharmaceutical product. In certain embodiments the positioning material may include a frit, a membrane, a screen, a fiber bed, any other mechanical support. The positioning material may either be a continuous structure or may be a patterned structure. For example, the positioning material may be a continuous porous plate, or may be in the form of a grid. In some embodiments, the positioning material itself may be configured to selectively retain one or more components from the pharmaceutical mixture. In one embodiment, the positioning material may retain the one or more components mechanically. For example, the positioning material may be a porous material having pores smaller than the particles of the component to be retained. For example, the pore size of the positioning material is in a range from about 5 micrometers to about 30 micrometers. In another embodiment, the positioning material may be configured to chemically retain the one or more components from the pharmaceutical mixture. The positioning material may be made from materials including but not limited to, hydrophobic and hydrophilic porous polymers including polyethylene and Teflon™ that may be used in medical applications without reacting with the medium or pharmaceutical product.

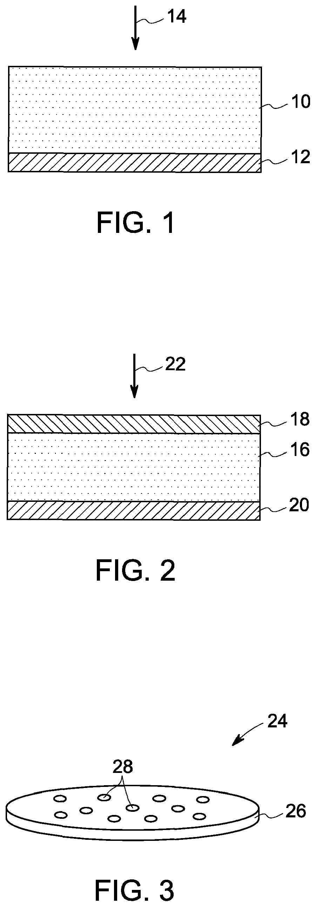

As will be described with regard to FIGS. 1-2 and 4-10, the resin and the positioning material may be arranged in several different configurations. In some embodiments, the resin may be configured for chemical retention of some components, while the positioning material may be configured for mechanical retention of same or different components. The resin and the positioning material may be complimentary to each other in terms of retaining the one or more components, for example the resin may retain the soluble undesirable components from the mixture and the positioning material may retain insoluble components from the mixture. Alternatively, the resin and the positioning material may be configured to retain the same one or more components. In these embodiments, the positioning material may be used as a back up arrangement to retain the components left by the resin and visa versa.

In one embodiment, the pharmaceutical mixture may include species that are soluble in the medium. In this embodiment, the resin is configured to retain the soluble species. In another embodiment, the pharmaceutical mixture may include species that are insoluble in the medium. In this embodiment, the positioning material and/or the resin are configured to retain the insoluble species that are to be separated/extracted from the mixture. In another embodiments, some of the species may be soluble while others may be insoluble in the medium. In certain embodiments, the device is configured to retain both soluble and insoluble species from the pharmaceutical mixture. In this embodiment, the positioning material and/or the resin is configured to retain some of the insoluble species while the resin is configured to retain the soluble species to be separated from the pharmaceutical mixture. Further, in some embodiments, the solubility of some of the species in the pharmaceutical mixture may vary with time as a function of the conditions of the media including pH, polarity, concentration, and temperature. In these embodiments, the resin or the positioning material or both may be configured to retain the species having time varying solubility. In these embodiments, the positioning material and/or the resin retain the insoluble components during certain phases of the filtration process. As the solubility of the components is modified by the properties of the solution, the insoluble materials become soluble and re-enter the solution. These resolubilized components are then retained on the resin. For example, in case of a mixture of electron paramagnetic agent (EPA) and pyruvic acid, the EPA is initially insoluble and retained by the positioning material. Subsequently, when the EPA becomes soluble, the EPA may be removed by the activated resin. An advantage of this method is that the dynamic solubility of the EPA may be controlled by modifying the composition of the medium and/or varying the dissolution conditions which include one or more of a pressure and temperature, for example. The volume (or weight) ratio of the resin and the positioning material are selected for the given application or intended use, In one example, a pharmaceutical mixture in the range of about 1 gram to about 2 grams requires a resin in a range from about 3 grams to about 10 grams.

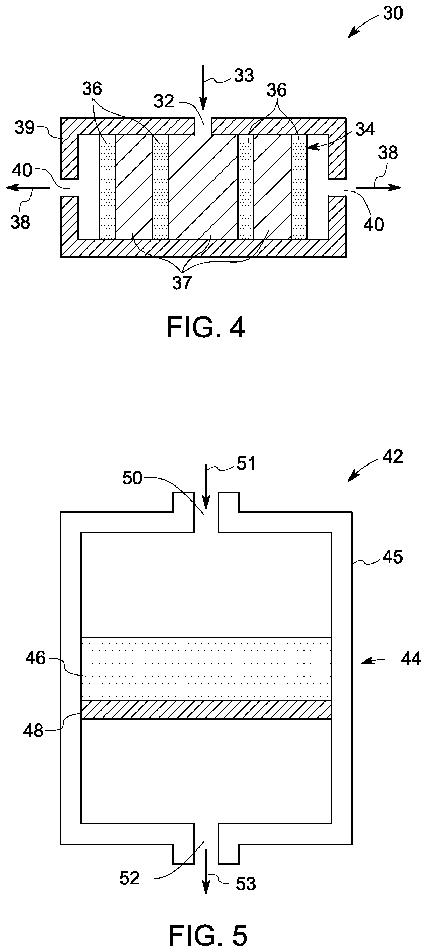

Further, the device also includes a filter body to house the arrangement of the resin and the positioning material. The filter body may be of various shapes and sizes depending on the requirements of filtration. For example, the filter body may have provisions for mixing a medium to the pharmaceutical product to form a pharmaceutical mixture. In another embodiment, the filter body may have provisions for varying the flow of a mixture of the pharmaceutical product to the resin. As will be described with regard to FIGS 4-10, in some embodiments, the filter body may have a plurality of arrangements of resin and positioning materials. In these embodiments, each of the plurality of arrangements may have the same configuration of the resin and the positioning material. Alternatively, some or all of the plurality of arrangements may have configurations that are different from others.

In some embodiments, the device may also include a diffuser configured to distribute the mixture into a plurality of streams. The diffuser is used before the positioning material to evenly distribute the mixture over the entire face of the positioning material. The diffuser may be employed to homogenize the mixture prior to the mixture being filtered by the resin. As with the positioning material, the diffuser may be made of a material that is inert to any solvents, medium, or pharmaceutical components in the mixture.

In certain embodiments, the device as disclosed is described in context of nuclear magnetic resonance (NMR) analysis, and particularly with regard to magnetic resonance imaging (MRI). It should be noted that the techniques described herein may be applied in various other systems other than the MRI. Also, the device may be employed to use pharmaceutical compounds other than the one employed in MRI.

MRI and NMR spectroscopy lack sensitivity due to the normally very low polarization of the nuclear spins of the samples used. A number of techniques exist to improve the polarization of nuclear spins in the solid phase. These techniques are known as hyperpolarization techniques and lead to an increase in sensitivity. As used herein, the term "polarize" or "polarization" refers to the modification of the physical properties of a solid material for further use in MRI. Further, as used herein, the term "hyperpolarized" refers to polarized to a level over that found at room temperature and 1T, which is further described in US 6,466,814. In hyperpolarization techniques, a sample of an imaging agent, for example ¹³C pyruvate or another similar polarized metabolic imaging agent, is introduced or injected into the subject being imaged. Typically, ¹³C pyruvate is mixed with tris(8-carboxyl-2,2,6,6-tetra(2-(1-methoxy-2,2-d2-ethyl))-benzo[1,2-d:4,5-d'] bis(dithiole-4-yl)methyl sodium salt, more commonly known as electron paramagnetic agent (EPA) to enhance the polarization of ¹³C. As will be appreciated, EPA, a processing agent needs to be removed from the pharmaceutical product before administering the product in the subject.

In one example, where a 100 ml of pharmaceutical product is being formed, approximately 10 to 50 micromoles of EPA are added to the pyruvic acid. Assuming no filtration, this amount of EPA results in an EPA concentration of 100 to 500 micro-molars in the dissolution product. It is desirable to have EPA concentration less than or equal to permitted levels in the dissolution product, corresponding to a high filtration efficiency. For example, the filtration device may be greater than about 90 percent. Accordingly, it is required to bring down the concentration of EPA to an allowable limit in the product following polarization and dissolution of the frozen pyruvic acid sample. In the presently contemplated embodiment, EPA is at least partially filtered out using the filtration devices of the present technique.

EPA exhibits solubility that varies with pH. At pH less than 4, EPA is predominantly insoluble and forms particulate with diameter greater than or equal to 10 microns. Above pH 4, EPA is soluble in aqueous solutions.

In certain embodiments, pyruvic acid and EPA are initially located in a small vial in a frozen state. An aqueous solution, known as the dissolution media, is used to melt the pyruvic acid and EPA and carry it through a filter to a final receiving vessel. This dissolution media can also be used to modify the pH of the product as needed. Because of the time associated with the melting process, the liquid arriving at the filter can at any given time have varied acid and EPA concentration as well as pH ranging from 2-12. These concentration and pH profiles vary as a function of time during the dissolution process.

In methods and devices in accordance with the present invention, a solid sample of the frozen pyruvic acid and EPA can be polarized while in the solid phase by any appropriate known method, e.g. brute force polarization, or dynamic nuclear polarization, while being maintained at a low temperature (e.g. under 100 K) in a strong magnetic field (e.g. 1-25 T). After the solid sample has been polarized, it is melted with a minimum loss of polarization. In the following the expression "melting means" will be considered to mean the following: a device capable of providing sufficient energy to the solid polarized sample to melt it or otherwise bring the polarized sample into solution for introduction into the subject being imaged. As used herein, the term "solid" refers to solid materials; semi-solid materials or any combination thereof provided the material requires some transformation to attain a liquid state suitable for introduction into a subject being imaged.

Turning now to FIG. 1, a resin is disposed in the form of a layer 10. A positioning material 12 is disposed on one side of the resin 10 in a direction of a flow of a stream of the mixture as represented by arrow 14. Typically, the positioning material 12 is configured to provide mechanical support to the resin 10 to at least partially retain the resin 10 in its position. The positioning material 12 may be a continuous layer. Although not illustrated, the positioning material 12 may include patterned structure. For example, the positioning material 12 may be a grid, or a plurality of rods, a frame. As described above, in some embodiments, the positioning material 12 may also be employed to assist the resin in selectively retaining one or more components from the mixture.

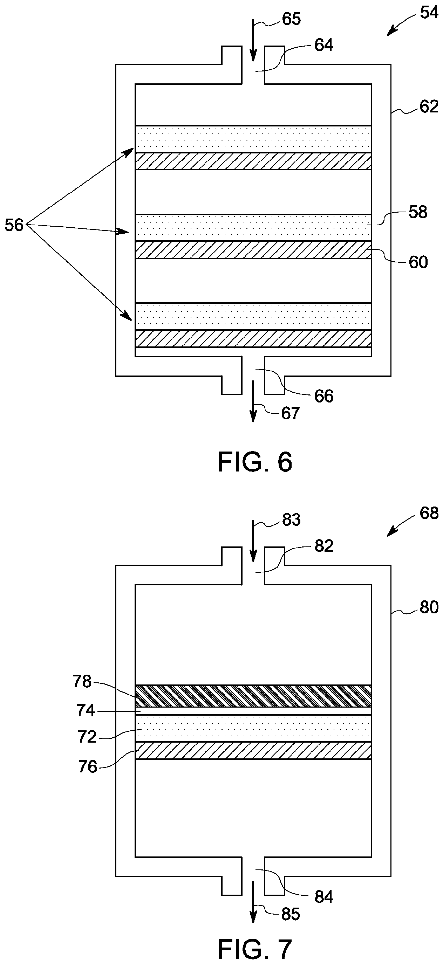

FIG. 2 illustrates an alternate embodiment of the arrangements of the resin and positioning material of FIG. 1. In the illustrated embodiment, the resin 16 includes positioning materials 18 and 20 on either side of the resin 16 in the direction of flow of the mixture as illustrated by the arrow 22. As described with regard to FIG. 1, the positioning materials 18 and 20 may have solid or non-solid patterns. Further, the configuration of the illustrated embodiment may be repeated. In other words, the device may employ a stack having a plurality of resins 16, disposed between a plurality of positioning materials 18 and 20. In one embodiment, the positioning material 18 may be configured to act as a diffuser. Generally, the resin is tightly packed against the positioning material. However, a distance between the top of the filter body and the diffuser/positioning material may be desired in some cases. For example, in case of a filter body employing a plurality of resin-positioning material pairs, the distance between the positioning material of one pair and the resin of the adjacent pair may allow for remixing of product prior to the next resin bed. This approach may also be beneficial in situations where the previous resin bed has been locally saturated with the chemical component (e.g., EPA) to be filtered causing bleed through while other areas remain unsaturated. This situation may arise if the diffuser did not evenly distribute flow, for example. Hence, the spaces allows for redistribution of the mixture. As will be described with regard to FIG. 7, a separate diffuser plate may also be applied in addition to the positioning materials 18 and 20.

FIG. 3 illustrates a perspective view of a diffuser 24 that may be employed in place of or in addition to the positioning material 18. The diffuser 24 includes a plate 26 having a plurality of through holes 28. The mixture is distributed into a plurality of streams, which pass through the holes 28. The streams eventually unite within the resin, for example resin 16, as shown in FIG. 2.

FIG. 4 illustrates an exemplary filtration device 30 having an inlet 32, the direction of flow of the mixture at the inlet 32 is represented by the arrow 33. The device 30 includes a layered structure 34 having alternate arrangements of layers of positioning material 36 and resins 37. The direction of flow of the filtered mixture is illustrated by arrows 38 exiting out of the filter body 39 through the outlets 40.

FIG. 5 illustrates a filtration device 42. The filtration device 42 may employ any combination of the configurations or parts of FIGS. 1-2, or their derivatives. In the presently contemplated embodiment, the device 42 includes an arrangement 44 having a resin 46 disposed on a positioning material 48. The device 42 further includes a filter body 45 having an inlet 50 and an outlet 52 for the mixture to enter and exit the device 42,as represented by arrows 51 and 53, respectively.

FIG. 6 illustrates an alternate embodiment of the device 42 of FIG. 5. In the illustrated embodiment, the device 54 includes a plurality of arrangements 56 disposed within the body 62. Each of the plurality of arrangements 56 may or may not be identical. In the illustrated embodiment, each of the configurations 56 includes a resin 58 and a positioning material 60. Further, the body 62 includes an inlet 64 and an outlet 66 for entry and exit of the mixture as represented by arrows 65 and 67, respectively.

FIG. 7 illustrates yet another embodiment of the devices 42 and 54 of FIGS. 5 and 6, respectively. In the illustrated embodiment, the device 68 employs a resin 72, positioning materials 74 and 76. The device 68 further includes a diffuser 78. Further, the device 68 includes a filter body 80 having inlet 82 and an outlet 84, for directing the flow of the mixture as illustrated by arrows 83 and 85, respectively.

Referring further to FIG. 7, in one embodiment, the pharmaceutical mixture has a time varying pH gradient, such as found in pyruvate and EPA mixture. Initially, the pH of the mixture tends to be acidic and with time the pH increases and shifts towards neutral or basic. In this embodiment, a filter containing a resin 72 disposed between entrance and exit positioning materials 74 and 76 is employed. Additionally, a diffuser 78 is used before the entrance positioning material 74 to evenly distribute the liquid over the entire surface of the resin 72. In this embodiment, the pyruvic acid and EPA are dissolved with an aqueous solution that has a basic pH, such as water containing sodium hydroxide. In addition, other pH modifiers or buffers may also be added to the pharmaceutical mixture. In a further embodiment, where the pharmaceutical mixture comprises pyruvic acid and EPA undergoing hyperpolarizatoin, as the pyruvic acid melting process commences, the ratio of acid to base in the solution above the frozen pyruvic acid and EPA is dominated by the acid and therefore the pH of the solution is acidic. Under this condition, the solution that is initially filtered has a pH < 4, making EPA insoluble. At this time the EPA, being an insoluble particulate, is trapped on the entrance positioning material 74 or on the front surface of the resin bed 72. At the same time the pyruvic acid aqueous solution passes through the entrance positioning material 74, enters the resin bed 72, wetting and thus activating the resin 72, passes through the exit positioning material 76 and exits the filter. As the dissolution continues, the ratio of acid to base in the vial decreases, causing a shift in the pH of the liquid leaving the vial to more neutral or basic pH. As the mixture arrives at the filter, EPA, either in the liquid or having been previously trapped on the entrance positioning material 74 or front the resin bed 72, becomes soluble and enters the resin bed 72. Within the resin bed 72, the soluble EPA is retained on the activated resin 72, while the pyruvic acid and sodium pyruvate pass through the filter to the receiving vessel.

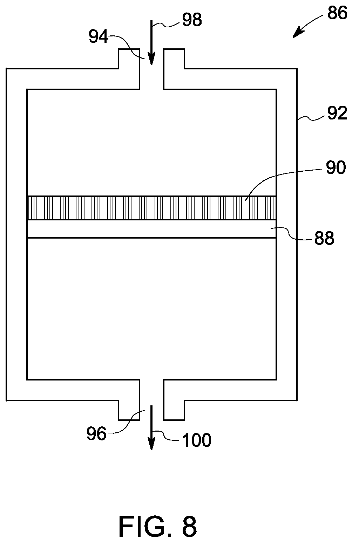

Turning now to FIG. 8, a filtering device 86 includes a positioning material 88 and a diffuser 90 disposed over the positioning material 88 in the direction of flow of the mixture. The device 86 does not include a resin. Such a device 86 may be employed in cases where the pharmaceutical mixture contains only insoluble species that need to be separated out from the pharmaceutical product. The device further includes an inlet 94 and an outlet 96 and the direction of flow of the mixture in the device 86 is illustrated by arrows 98 and 100.

In the illustrated embodiment of FIG. 8, the pH of the mixture may be in the acidic range. In one embodiment, a device 68 containing only positioning material 88 without a resin bed is employed. A diffuser 90 is used before the positioning material 88 to evenly distribute the mixture over the entire face of the positioning material 88. In this embodiment, the pyruvic acid and EPA are dissolved with an aqueous solution that has a neutral pH, such as pure water. Under this condition, the solution that is initially filtered has a pH < 4, making EPA insoluble. At this time the EPA, being an insoluble particulate, is trapped on the positioning material 88. At the same time the pyruvic acid aqueous solution passes through the positioning material 88. Subsequently, the pyruvic acid is neutralized to form sodium pyruvate within a receiving vessel.

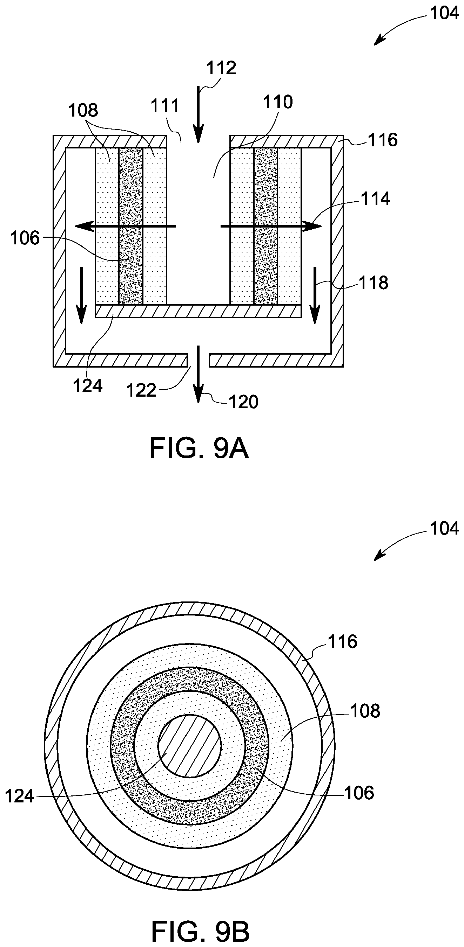

Turning now to FIG. 9A, a radial filtration device 104 is illustrated. FIG. 9B illustrates the top view of the device 104. The resin material 106 and the positioning material 108 are co-centrically arranged around the passage 110 for mixture entry through the entry 111. Such a filtration device 104 may be employed in filtration applications where the mixture enters the device 104 at a high flow rate. Also, the filtration device 104 may be employed for large-scale filtration applications. The mixture enters the passage 110 in a direction illustrated by the arrow 112, and is distributed in radial direction (as illustrated by the arrows 114) owing to the incoming mixture pressure and the arrangement of the resin and the positioning materials. The filtered mixture then percolates through the resin material 106 and the positioning material 108 to enter the passage between the walls of the filter body 116 and the arrangement of and the resin material 106 and the positioning material 108 as illustrated by the arrows 118. The mixture then exits (arrow 120) through the exit 122 of the filter body 116 by passing through a passage between the walls of the filter body and the support plate 124 holding the arrangement of the resin material 106 and the positioning material 108. Although not illustrated, in an alternate arrangement, the filtering device may also function if the mixture path is represented by reversing the direction of arrows 112, 114, 118 and 120 by 180°. That is, the mixture to be filtered is entered into filter body 116 through location 122 and moves through the passage between the walls of the filter body 116 and the arrangement of the resin material 106 and the positioning material 108. The mixture then enters the arrangement of the resin material 106 and the positioning material 108 to be filtered. The filtered mixture is collected at location 111.

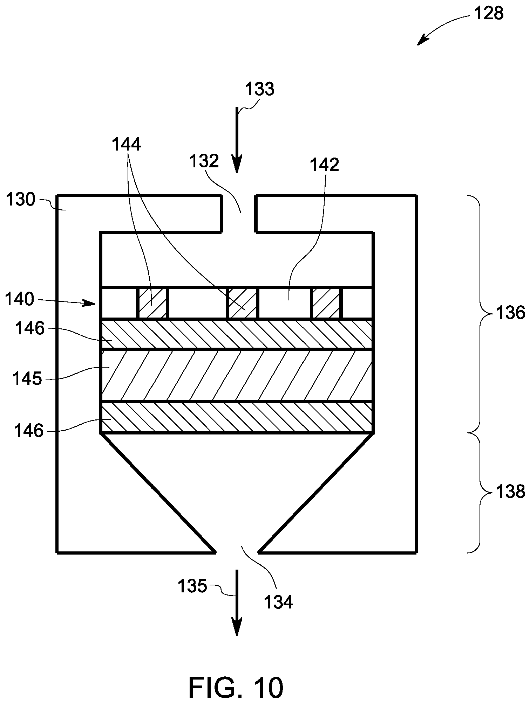

Turning now to FIG. 10, a filtering device 128 having a filter body 130, an inlet 132 and an outlet 134 is illustrated. Arrows 133 and 135 represent the direction of flow of the mixture. A portion 136 of the filter body 130 has a cylindrical cross-section, while the rest of the portion 138 of the filter body 130 has a conical shape. The conical shape of the portion 138 facilitates easy exit of the mixture from the filter body 130 following filtration. The filtration device 128 further includes a diffuser 140 having a substrate 142 with holes 144. The filtration device 128 also includes a resin material 145 disposed between two positioning materials 146.

In certain embodiments, an unrefined pharmaceutical product is mixed with a basic medium, a buffer and a chelator to form a filtering mixture. For example, the unrefined pharmaceutical product may include pyruvic acid, and electron paramagnetic agent. The basic medium may include sodium hydroxide, the buffer may include tris(hydroxymethyl)-aminomethane (TRIS). The presence of chelator may facilitate separation of metal ions from the mixture. The mixture is then entered into the filter body via the inlet. Once inside the filter body, the mixture passes through the resin and the positioning material, and exits the device via the outlet.

In certain embodiments, the filtration device, such as the device 42 or 54 may be employed in nuclear magnetic resonance (NMR) analysis, particularly to nuclear magnetic resonance imaging (MRI) and analytical high-resolution NMR spectroscopy. For example, the device may be employed for filtration of polarizing materials prior to their administration in the body of a patient for the purpose of magnetic resonance imaging (MRI).

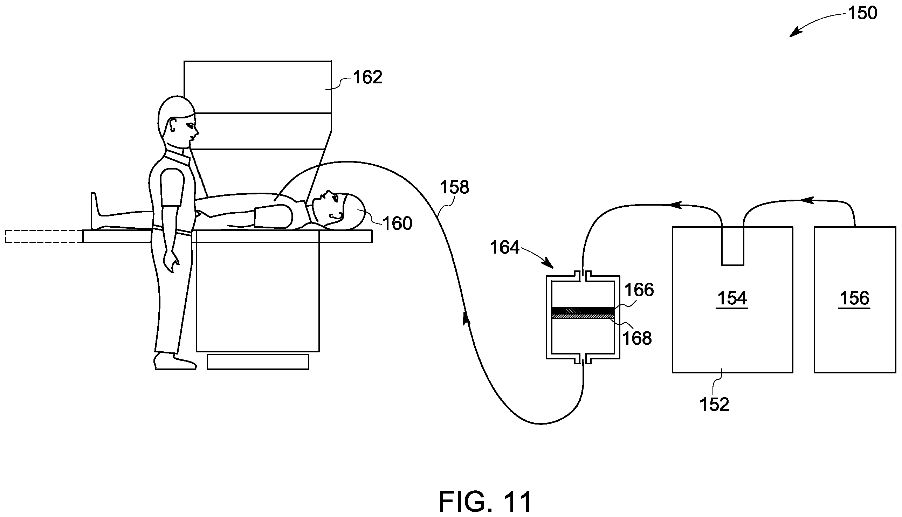

Referring to FIG. 11, an exemplary system 150 is shown for producing hyperpolarized samples for use in a MRI device and includes a cryostat 152 and polarizing subsystem 154 for processing material from a container 156 and resulting in the hyperpolarized material. A material delivery line 158 is used to deliver the hyperpolarized material to subject 160 within MRI scanner 162.

In the embodiment shown in FIG. 11, the hyperpolarized samples are used in an in vivo imaging application. It is to be appreciated that hyperpolarized samples may also be produced using the methods and techniques described below for Nuclear Magnetic Resonance (NMR) analysis. A filter 164 is attached to the delivery line 158 and positioned between the cryostat 152 and the subject 160 to filter the hyperpolarized material, before it is administered in the subject 160. The filter 164 includes a resin material 166 and a positioning material 168, as discussed above with regard to FIGS. 4-10. Although not illustrated, in some embodiments, two or more filters, such as filters 164 may be employed in series arrangement in the system 150. In these embodiments, the plurality of filters 164 may be arranged such that an output from one filter serves as an input for the adjacent filter in the direction of the subject 160. Each of the filters of the two or more filters may have either the same or different configurations, compositions of the resin, the positioning material and/or a diffuser.

While only certain features of the invention have been illustrated and described herein, many modifications and changes will occur to those skilled in the art. It is, therefore, to be understood that the appended claims are intended to cover all such modifications and changes as fall within the true spirit of the invention.

## Claims

1. A method of filtering an unrefined pharmaceutical product, comprising:
mixing a buffer, a chelator, and one of an acidic medium, a basic medium, or a neutral medium, with the unrefined pharmaceutical product to form a filtering mixture, wherein the unrefined pharmaceutical product comprises pyruvic acid and an electron paramagnetic agent; and
passing the filtering mixture through a device for dynamic filtration of a pharmaceutical product, comprising
a resin configured to be activated during the filtration process by the medium of the mixture to be filtered; in its activated state, said resin is configured to filter out the one or more undesired components from the mixture of the pharmaceutical product by retaining the undesired components and allowing the rest of the mixture to pass through the resin;
at least one positioning material disposed adjacent to the resin, wherein the positioning material is configured to provide mechanical support to the resin to at least partially retain the resin in position; and
a filter body to house the resin and the positioning material; wherein
the resin and/or the positioning material of the device are configured to retain insoluble species while the resin is configured to retain soluble species that are to be separated from the mixture.

2. The method of claim 1, wherein the medium of the mixture is aqueous based.

3. The method of claim 2, wherein the aqueous based medium changes dynamically from acidic to basic, or basic to acidic, or from a neutral pH to either basic or acidic conditions or from an acidic or basic condition to a neutral pH.

4. The method of claim 1, wherein the solution that is initially filtered has a pH < 4.

5. The method of claim 1, wherein the unrefined pharmaceutical product comprises species having time varying solubility in the mixture; said species being insoluble under acidic conditions and soluble under basic conditions; said insoluble species being retained on the positioning material and said soluble species being filtered by the resin.

6. The method of claim 1, wherein the unrefined pharmaceutical product comprises both soluble and insoluble species.

7. The method of claim 1, wherein a flow rate of the mixture at the filtering device is in a range from about 3 mL/s to about 12 mL/s.

8. The method of claim 1, wherein the filtering device further comprises a diffuser to temporarily distribute the mixture comprising the pharmaceutical products into a plurality of streams.

9. The method of claim 1, wherein the resin is adapted for surface modifications, wherein the surface modifications include a non-polar functionality or a combination of a non-polar and a polar functionality.

10. The method of claim 1, wherein a pore size of the resin is in a range from about 60 A° to about 4000 A°.

11. The method of claim 1, wherein the at least one positioning material is configured to retain one or more components from the mixture comprising the pharmaceutical product.

12. The method of claim 1, wherein the positioning material is configured to retain insoluble components from the mixture.

13. The method of claim 1, wherein the at least one positioning material is disposed on one side, or on either side, of the resin in a direction of a flow of a stream of the mixture.

14. The method of claim 1, wherein the at least one positioning material comprises a frit, a membrane, a fiber bed, a screen, a support, or combinations thereof.

## Patentansprüche

1. Verfahren zur Filtration eines unraffinierten pharmazeutischen Produkts, umfassend:
Mischen eines Puffers, einer chelatbildenden Verbindung und eines von einem sauren Medium, einem basischen Medium oder einem neutralen Medium mit dem unraffinierten pharmazeutischen Produkt zur Bildung einer Filtermischung, wobei das unraffinierte pharmazeutische Produkt Brenztraubensäure und ein paramagnetisches Elektronenmittel umfasst; und Durchleiten der Filtermischung durch eine Einrichtung zur dynamischen Filtration eines pharmazeutischen Produkts, umfassend
ein Harz, das dafür ausgeformt ist, während des Filtrationprozesses durch das Medium der zu filternden Mischung aktiviert zu werden; in seinem aktivierten Zustand ist das Harz dafür ausgeformt, den einen oder mehrere unerwünschte Bestandteile aus der Mischung des pharmazeutischen Produkts durch Festhalten der unerwünschten Bestandteile auszufiltern und den Rest der Mischung durch das Harz passieren zu lassen;
mindestens ein Positionierungsmaterial, das neben dem Harz angeordnet ist, wobei das Positionierungsmaterial dafür ausgeformt ist, mechanische Unterstützung für das Harz bereitzustellen, um das Harz zumindest teilweise in der richtigen Position zu halten; und
einen Filterkörper zum Aufnehmen des Harzes und des Positionierungsmaterials; wobei
das Harz und/oder das Positionierungsmaterial der Einrichtung dafür ausgeformt ist bzw. sind, unlösliche Spezies festzuhalten, während das Harz dafür ausgeformt ist, lösliche Spezies festzuhalten, die von der Mischung getrennt werden sollen.

2. Verfahren nach Anspruch 1, wobei das Medium der Mischung auf Wasser basiert.

3. Verfahren nach Anspruch 2, wobei sich das wasserbasierte Medium dynamisch von sauer zu basisch oder basisch zu sauer oder von einem neutralen pH-Wert zu entweder basischen oder sauren Zuständen oder von einem sauren oder basischen Zustand zu einem neutralen pH-Wert ändert.

4. Verfahren nach Anspruch 1, wobei die eingangs gefilterte Lösung einen pH-Wert < 4 aufweist.

5. Verfahren nach Anspruch 1, wobei das unraffinierte pharmazeutische Produkt Spezies mit zeitvariierender Löslichkeit in der Mischung umfasst; welche Spezies unter sauren Bedingungen unlöslich und unter basischen Bedingungen löslich sind; wobei die löslichen Spezies auf dem Positionierungsmaterial festgehalten und die löslichen Spezies durch das Harz gefiltert werden.

6. Verfahren nach Anspruch 1, wobei das unraffinierte pharmazeutische Produkt sowohl lösliche als auch unlösliche Spezies umfasst.

7. Verfahren nach Anspruch 1, wobei eine Strömungsgeschwindigkeit der Mischung an der Filtereinrichtung im Bereich von ca. 3 ml/s bis ca. 12 ml/s liegt.

8. Verfahren nach Anspruch 1, wobei die Filtereinrichtung weiter einen Diffusor zur temporären Verteilung der Mischung umfassend das pharmazeutische Produkt in eine Mehrheit von Strömen umfasst.

9. Verfahren nach Anspruch 1, wobei das Harz für Oberflächenmodifikationen angepasst wird, wobei die Oberflächenmodifikationen eine nicht-polare Funktionalität oder eine Kombination aus einer nicht-polaren und einer polaren Funktionalität umfassen.

10. Verfahren nach Anspruch 1, wobei eine Porengröße des Harzes im Bereich von ca. 60 A° bis ca. 4000 A° liegt.

11. Verfahren nach Anspruch 1, wobei das zumindest eine Positionierungs-material dafür ausgeformt ist, einen oder mehrere Bestandteile aus der Mischung umfassend das pharmazeutische Produkt festzuhalten.

12. Verfahren nach Anspruch 1, wobei das Positionierungsmaterial dafür ausgeformt ist, unlösliche Bestandteile aus der Mischung festzuhalten.

13. Verfahren nach Anspruch 1, wobei das zumindest eine Positionierungs-material auf einer Seite, oder auf beiden Seiten, des Harzes in einer Richtung einer Strömung der Mischung angeordnet ist.

14. Verfahren nach Anspruch 1, wobei das zumindest eine Positionierungs-material eine Fritte, eine Membran, ein Faserbett, ein Sieb, eine Unterstützung oder Kombinationen davon umfasst.

## Revendications

1. Procédé de filtrage d'un produit pharmaceutique non raffiné, comprenant les étapes consistant à:
mélanger un tampon, un agent chélatant, et l'un d'un milieu acide, un milieu basique ou un milieu neutre, avec le produit pharmaceutique non raffiné pour former un mélange de filtration, dans lequel le produit pharmaceutique non raffiné comprend de l'acide pyruvique et un agent paramagnétique d'électrons; et
faire passer le mélange de filtration à travers un dispositif de filtrage dynamique d'un produit pharmaceutique, comprenant
une résine configurée de manière à être activée lors du procédé de filtrage par le milieu du mélange à filtrer; dans son état activé, ladite résine est configurée de manière à extraire l'un ou plusieurs composants non désirés par filtrage du mélange du produit pharmaceutique en retenant les composants non désirés et permettant au reste du mélange de passer à travers la résine;
au moins un matériau de positionnement disposé de manière adjacente à la résine, ledit matériau de positionnement étant configuré de manière à fournir un support mécanique à la résine pour retenir au moins partiellement la résine en position; et
un corps de filtre pour loger la résine et le matériau de positionnement; dans lequel
la résine et/ou le matériau de positionnement du dispositif sont configurés de manière à retenir des espèces insolubles tandis que la résine est configurée de manière à retenir des espèces solubles à séparer du mélange.

2. Procédé selon la revendication 1, dans lequel le milieu du mélange est à base aqueuse.

3. Procédé selon la revendication 2, dans lequel le milieu à base aqueuse change dynamiquement à partir d'acide en basique, ou à partir de basique en acide, ou à partir d'un pH neutre soit en conditions basiques soit en conditions acides, ou à partir d'une condition acide ou basique en un pH neutre.

4. Procédé selon la revendication 1, dans lequel la solution qui est initialement filtrée présente un pH < 4.

5. Procédé selon la revendication 1, dans lequel le produit pharmaceutique non raffiné comprend des espèces ayant une solubilité variant en temps dans le mélange; lesdites espèces étant insolubles dans des conditions acides et solubles dans des conditions basiques; lesdites espèces insolubles étant retenues sur le matériau de positionnement et lesdites espèces solubles étant filtrées par la résine.

6. Procédé selon la revendication 1, dans lequel le produit pharmaceutique non raffiné comprend des espèces à la fois solubles et insolubles.

7. Procédé selon la revendication 1, dans lequel un débit d'écoulement du mélange au niveau du dispositif de filtrage se trouve dans une plage d'environ 3 mL/s à environ 12 mL/s.

8. Procédé selon la revendication 1, dans lequel le dispositif de filtrage comprend en outre un diffuseur pour répartir temporairement le mélange comprenant les produits pharmaceutiques en une pluralité de courants.

9. Procédé selon la revendication 1, dans lequel la résine est adaptée pour des modifications de surface, lesdites modifications de surface comprenant une fonctionnalité non polaire ou une combinaison d'une fonctionnalité non-polaire et polaire.

10. Procédé selon la revendication 1, dans lequel une taille de pores de la résine est dans une plage comprise entre environ 60 A° et environ 4000 A°.

11. Procédé selon la revendication 1, dans lequel l'au moins un matériau de positionnement est configuré de manière à retenir un ou plusieurs composants du mélange contenant le produit pharmaceutique.

12. Procédé selon la revendication 1, dans lequel le matériau de positionnement est configuré de manière à retenir des composants insolubles à partir du mélange.

13. Procédé selon la revendication 1, dans lequel l'au moins un matériau de positionnement est disposé sur un côté, ou de part et d'autre, de la résine dans une direction d'un écoulement d'un courant du mélange.

14. Procédé selon la revendication 1, dans lequel l'au moins un matériau de positionnement comprend une fritte, une membrane, un lit de fibres, un écran, un support, ou des combinaisons de ceux-ci.
